# EUROPEAN PATENT SPECIFICATION

(11) **EP 1 027 870 B1**
(45) Date of publication and mention of the grant of the patent: **30.03.2005**
(21) Application number: 00200101.4
(22) Date of filing: 12.01.2000
(51) Int. Cl.: A61F 2/06

(54) **Expandable intraluminal endoprosthesis**
Intraluminale aufweitbare Endoprothese
Endoprothèse intraluminale expansible

(30) Priority: 12.01.1999 EP 99200067
(43) Date of publication of application: 16.08.2000
(73) Proprietor: Orbus Medical Technologies, Inc., Ft Lauderdale, Florida 33309 (US)
(72) Inventor: Cottone, Robert J., Ft. Lauderdale, Florida 33309 (US); Becker, Gary J., Miami, Florida 33156 (US)
(74) Representative: Jilderda, Anne Ayolt

(56) References cited:
- EP-A- 0 884 029
- WO-A-98/40035
- DE-A- 19 722 384
- US-A- 5 810 872

## Description

The present invention relates to an expandable intraluminal endoprosthesis comprising a tubular member having a first and second end and a wall surface disposed between said first and second end, the wall having a first diameter in a first, unexpanded state which permits intraluminal delivery of the member into a lumen of a body passageway, particularly a blood vessel, and being capable of acquiring a second diameter in an expanded and deformed state upon the exertion of a radially outwardly extending force to expand the lumen of the body passageway. More particularly the invention relates to an expandable intraluminal vascular endoprosthesis which is especially useful for repairing or reconstructing blood vessels narrowed or occluded by a disease. Commonly this kind of medical device is referred to as vascular stent or graft.

Stents are prosthetic devices which are implanted inside a lumen in order to provide support for its wall and to assure an undisturbed flow through the lumen. This is particularly important in the field of angioplasty which is concerned with the repair and reconstruction of blood vessels. In that particular field stents are implanted within the vascular system to reinforce collapsing, partially occluded, weakened, or abnormally dilated sections of blood vessels. More generally, however, stents can be used inside the lumen of any physiological conduit or duct including the arteries, veins, bile ducts, the urinary tract, alimentary tracts, the tracheobronchial tree, a cerebral aqueduct and the genitourinary system. Moreover stents can be used inside lumina of animals besides humans.

Generally two types of stents may be distinguished. First there are self-expandable stents which automatically expand once they are released to assume a permanent deployed, expanded state. The outwardly extending force necessary for its deployment is provided by the spring force of the material used and is accordingly inherently available within the device itself. These stents expand to a defined diameter but are unable to remodel the true vascular anatomy over lengths greater than 2 cm. Their drawback is that the physician needs to place the right device and thereby has to rely on information derived from fluoro and angiographic equipment. However, the same spring force which is responsible for the deployment of the device offers it a relatively large hoop strength, i.e. the ability to withstand radial forces which are exerted on it from the outside.

A second type of stent concerns the so-called balloon expandable stent which generally involves a tubular member capable of receiving a balloon of a balloon-tipped catheter by means of which it may be deployed. A common procedure for implanting a balloon-expandable stent in a blood vessel involves mounting the stent in its unexpanded, crimped state on a balloon-tipped catheter of a suitable delivery system. The catheter is then slipped through an incision in the vessel wall and down the length of the vessel until it is positioned to bridge the diseased or narrowed portion of the vessel. The stent is then expanded with the aid of the balloon catheter against the internal wall of the vessel. This may be done after the vessel has been predilated and it has been determined that a stent is necessary. Alternatively the vessel could be dilated by the stent itself while the latter is expanded by means of the balloon. It both cases the stent will maintain its deployed, expanded form once the balloon is evacuated and the catheter retracted again in order to provide a permanent support for the blood vessel concerned.

A wide overview of vascular stents which are nowadays available is given in the Handbook of Coronary Stents by Patrick W. Serruys et al. of the Rotterdam Thoraxcentre Interventional Cardiology Group. This overview describes at page 21 ff. the so called Palmaz-Schatz™ stent as the gold standard in the field of stents. This stent concerns a number of consecutive slotted tubes of stainless steel which are mutually connected by means of one or more bridges. Although this stent is most widely used and tested in practice, having been implanted in over 600000 patients all over the world, it still suffers from a number of drawbacks. The main drawbacks have to do with the stent-to-vessel-ratio uniformity and crimped as well as deployed flexibility. The stent-to-vessel-ratio involves the degree to which the vessel is supported by the stent in its expanded state and should not only be high, but preferably also uniform throughout the length of the stent. However, due to the inevitable bridges between adjacent tubes of the Palmaz-Schatz™ stent, there will be a bare area between adjacent segments of the stent once it has been deployed, giving rise to a decreased and even poor stent-to-vessel-ratio at these locations. The other drawback concerns the rather high rigidity of the stent segments in their crimped and deployed state. As a consequence, the stent has only a limited flexibility which hinders the delivery of the stent to its intended position inside the body. The poor deployed flexibility of this stent gives rise to a straightening of the vessel over segments longer than typically 2 cm which appears to be a primary cause for late term restenosis of the stented area. Typically this may occur about 6 months after the stent implantation.

A balloon expandable stent with a highly uniform stent-to-vessel ratio as well as an excellent flexibility in its crimped state is described at page 63 ff. of the same reference and concerns the Cordis Coronary Stent. This device is composed of a single piece of tantalum (Ta) wire. The wire is wrapped to form a continuous sine wave and helically wound along a longitudinal axis. Both ends of the wire are weld terminated. A similar device was presented at the annual symposium of the Radiological Society of North America (RSNA) 11/95. This peripheral stent embodiment incorporates intermediate welds, patterned through the length of the stent. This device contains adjacent helical turns of the wire which are welded together at adjoining locations and exhibits a highly regular distribution of the wire along the length of the device. Its properties, such as crimped profile and stent-to-vessel ratio, are uniform over its length both in the crimped and deployed states. However, because of its constitution this device offers only a poor design freedom when it comes to tailoring the design to add specific functionality and remove certain drawbacks. Also the internal stress in the device once it has been wound hinders the provision of reliable welds between adjacent turns. Moreover, these welds as well as those to the ends of the wire, remain a weak point especially during expansion of the device.

A balloon expandable stent which combines a high degree of uniformity and flexibility with excellent design capabilities is described in European patent application EP 884.029. This device features a substantially continuous structure of mutually staggered undulations which has been separated from a tube wall. Said substantially continuous structure comprises at least one pattern which advances substantially helically along a longitudinal axis of said tubular body and comprises connection elements connecting adjacent undulations. The connection elements are an integral extension of the undulations which they connected.

Although the device of European patent application EP 884.029 provides excellent properties especially regarding its flexibility both in a compressed and deployed state, it may still be prone to damage when subjected to an external load. As such it may be less suitable for application in peripheral vessels and arteries, for instance those in the limbs and abdominal region of a human body, which lack substantial protection by the skeleton. It is an object of the present invention to provide an expandable intraluminal endoprosthesis of the kind referred to in the opening paragraph with an improved ability to withstand external forces such that it is suitable for application in said peripheral vessels.

To this end, an expandable intraluminal endoprosthesis of the type described in the opening paragraph is provided according to cl. 1. The additional material delivered by the connection strut gives the device more rigidity in the deployed state. This is especially predominant in a preferred embodiment of the endoprosthesis which according to the invention is characterized in that primary undulations are mutually interconnected by means of a number of connections elements which are regularly distributed over a helical turn of said at least one winding and in that a connection strut is present from each connection element within said turn to a subsequent connection element within said turn. In a sense, these connection struts between connection elements provide for a secondary scaffolding pattern in the deployed state of the device, additional to the primary scaffolding by the winding(s) of primary undulations. By placing the connection elements and the connection struts at regular intervals with respect to each other, this secondary scaffolding will consist of a continuous helical winding advancing in between the primary undulations. As a result the structure features an enhanced hoop strength and an improved stent-to-vessel ratio in its deployed state, which renders the device particularly suitable in peripheral vessels and arteries where it is likely to be subjected incidentally to considerable external loads.

In order to allow a large radial expandability as well as expanded flexibility, a special embodiment of the endoprosthesis according to the invention is characterized in that the connection strut comprises a sub-structure of mutually staggered secondary undulations, at least in a crimped state of the device, lying in between subsequent turns of primary undulations. Accordingly the sub-structure formed by these undulations may be stretched so as to give way to the radial expansion of the device as a whole. Moreover, the stretching capability of the secondary undulations offers an improved flexibility of the device both in a crimped as well as in a deployed state. The latter feature contributes to an excellent capability of the device to conform itself to the natural anatomy of the body lumen in which it is to be implanted.

A further special embodiment of the endoprosthesis according to the invention is characterized in that the secondary undulations have an amplitude substantial equal to half the longitudinal pitch of said turns. In this manner the second undulations bridge the gap between both windings of primary undulations at least to the largest possible extent, which gives rise to an optimal stent-to-vessel ratio in the expanded state.

Still a further special embodiment of the endoprosthesis according to the invention is characterized in said primary undulations have a mutual pitch which is at least locally substantially an integer multiple of the mutual pitch of said secondary undulations. Thus it is possible to arrange the secondary undulations in phase with the primary undulations in order to obtain a regular structure. This structural regularity translates to highly predictable expansion characteristics.

A further special embodiment of the endoprosthesis according to the invention is characterized in that the connection elements comprise at least one tertiary undulation which lies embedded in said substructure of mutually staggered secondary undulations of said connection struts, at least in said first, unexpanded state of the device. Like the secondary undulations, said tertiary undulation provides for en enhanced radial expandability of the device. By embedding the tertiary undulation in the same substructure which is formed by the second undulations a substantially continuous secondary scaffolding structure is realized in the device, interposed between the primary undulations.

A further embodiment of the endoprosthesis according to the invention is characterized in that at least a part of said wall of said tubular member comprises at least two substantially continuous windings of mutually staggered primary undulations, advancing mutually substantial parallel along the longitudinal axis of said tubular member, in that a first winding comprises said first and third undulation, in that a second winding comprises said second and fourth undulation, and in that the connection struts are interposed in between both said windings. This embodiment features a dual-helix structure provided by both said windings with interposed connection elements and connection struts. Upon expansion of this device the interposed substructure will reorientate itself with respect to the longitudinal axis of the device such that the connection elements will turn substantially traverse to said axis whereas the connection struts will assume a more parallel orientation with respect to said axis. As a result pairs of connection elements may form discrete ring like elements substantially traverse to the axis of the body interconnecting both windings, in the deployed state of the device. These ring like elements are themselves interconnected by substantially longitudinally orientated connection struts. These bonds together give rise to excellent scaffolding properties and stent-to-vessel ratio of the device once it has been deformed to an expanded state, whereas the structure remains extremely flexible both in a crimped as well as in an expanded state. The total structure as a result features an outstanding hoop strength and stent-to-vessel ratio in the deployed state without compromising its crimped and deployed flexibility.

In a further preferred embodiment the endoprosthesis according to the invention is characterized in that said structure comprises a substantial continuous filament which has been separated from a tube wall, in that said connection elements are an integral extension of the primary undulations which they interconnect and in that the connection strut is an integral extension of the connection elements thereby connected. The filament may be formed by applying computer controlled laser cutting or another high precision technique to a solid tube. Thus the device may be formed in any configuration without introducing any stress in the structure.

The invention will now be further elucidated with reference to an illustrative example and an accompanying drawing in which:
- figure 1: represents an isometric view of a centre part of an embodiment of the endoprosthesis according to the present invention; and
- figure 2: shows a plan view of the device of figure 1 when cut and folded open.
This figure is drawn purely schematically and not everywhere to scale. Instead, some dimensions may be exaggerated for the sake of clarity. Like elements are provided with same reference numerals as much as possible.

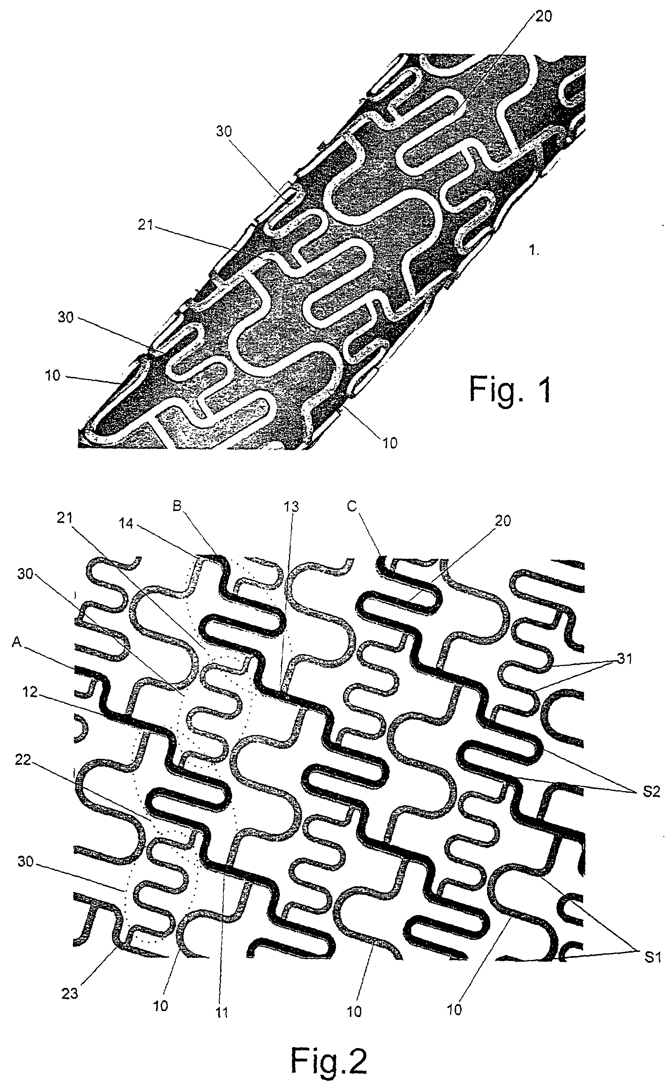

The intraluminal endoprosthesis shown in figure 1, a so called stent, comprises a substantially tubular member having a first and second end beyond the extremities of the drawing. A wall surface 1 is disposed in between these ends of the member of which a central module part is depicted in figure 1. Outside the drawing limits, this central module of repeating elements runs over in intermediate sections at both sides, which intermediate sections separate the centre module from end modules at opposite sides of the device. The basic difference between the different portions resides in the number of connection elements between adjacent undulations of the helical advancing pattern of the device, all as described in European patent application EP 884.029.

The wall surface 1 with its specific helical pattern has been cut or other wise liberated out of a solid tube wall of a tube of nitinol (NiTi) or any other suitable, bio-compatible material as described in European patent application EP 884.029. The wall surface of the stent is capable of acquiring a first crimped or unexpanded state which permits intraluminal delivery of the member into a lumen of a body passageway, particularly a blood vessel, as well as of second, expanded or deployed state upon exertion of a radially outwardly extending force to expand the lumen of the body passageway. The state shown corresponds to the first, crimped state, as it is manufactured. The radially outwardly extending force may be imposed on the device by means of a so called balloon tipped catheter which is received within the member and then inflated. Alternatively said force may be an intrinsic spring force of the device which is released by removing a sleeve from the device which initially covers it to confine the device in the crimped state. The first type of stent is generally referred to as a balloon-expandable stent, while the other stent is usually addressed as being self-expandable. The present embodiment focusses on the first type of stent, although it will be appreciated that the invention is also applicable to a self-expandable stent.

The structure making up the wall surface 1 of the member, at least in the depicted part of the device, essentially consists of a substantially continuous winding 10 of mutually staggered primary undulations 11-14, which advances substantially helically along a longitudinal axis of the tubular member. Adjacent primary undulations 11-14 from subsequent turns of said winding 10 are thereby mutually interconnected by means of connection elements 21,22,23 which bridge the gap between the respective turns. The connection elements 21,22,23 each comprise a s-curved bent 20 which greatly enhances the radial expandability of the device once it transforms from the crimped to the deployed state. Instead of connection elements 21,22,23 of the kind shown here, also otherwise devised connection elements may be used as described in the aforementioned co-pending application, just to suit the specific demands and desired characteristics of the device.

For more detail concerning the interconnections within the structure, reference is made to figure 2 which shows the structure of figure 1 in plan view after it has been cut and folded open. As can be seen, each time a first primary undulation 11 is connected to an associated second primary undulation 12 by means of a first connection element 21, while a third primary undulation 13 subsequent to the first one 11 is connected to a fourth primary undulation 14 subsequent to the second one 12 by means of a second connection element 22. To enhance the overall stent-to-vessel ratio of the device and, more importantly, its hoop strength, the first and second connection elements 21,22 are themselves interconnected by means of a connection strut 30 which lies interposed in between turns of the primary undulations 11-14. The connection strut 30 comprises a sub-structure of mutually staggered secondary undulations 31 which allow for a sufficient radial expandability of the device.

The second connection element 22 is likewise connected to a subsequent further connection element 23 by means of a further connection strut 30, such that the S-curved bents 20 of the connection elements 21,22,23 each form a kind of tertiary undulation lying embedded and integrated in said sub-structure of secondary undulations 31. This pattern continues along the helical path set by the winding 10 of primary undulations 11-14 such that a highly regular sub-structure is obtained of secondary undulations 31 and tertiary undulations 20 in between the primary undulations 11-14. In this case three connection elements 21,22,23 are applied per helical turn of the primary undulations 11-14. As a result three spines A,B,C may be distinguished in the structure advancing contra-helical to the winding 10 of primary undulations 11-14. These spiral spines A,B,C together with the winding 10 of primary undulations 11-14 create a double helix structure which results in an excellent hoop strength once it has been deployed. The winding 10 of primary undulations 11-14 is responsible for a major scaffolding within the structure while the spines A,B,C provide for minor scaffolding in the other helical direction. Both structures intersect at the primary undulations 11-14 interconnected by connection elements 21,22.

The mutual pitch sl of the primary undulations 11-14 is approximately equal to an integer multiple of the pitch s2 of the undulations in the substructure of secondary- and tertiary undulations 20,31 to nicely accommodate said sub-structure. Also the amplitude of the secondary undulations 31 is taken to be about half the longitudinal pitch p of the turns of primary undulations 11,14. This amplitude leads to an optimal stent to vessel ratio of the device as the gap in between said turns is almost entirely filled with the pattern defined by the substructure of secondary undulations.

Because the entire structure is cut from a tube wall by means of laser cutting or a similar technique, the structure in fact comprises a continuous, coherent filament. The connection elements are an integral extension of the primary undulations which they interconnect and likewise are the connection struts an integral extension of the connection elements thereby interconnected. The pattern, width and form of said filament may be precisely controlled and is realized by means of a computer aided technique. A designer has hence an extremely great freedom of tailoring the device to suit the desired characteristics without introducing stress or other mechanical compromises in the structure. As such the connection struts 30 have been given a smaller filament width than the primary undulations 11-14, in order to gain flexibility, both in the crimped as in the expanded state. In this example, these widths are respectively approximately 0,15 and 0,20 mm. The connection elements 21,22,23 have an intermediate width of about 0,18 mm.

In practice the device is positioned on the balloon of a balloon tipped catheter and guided with the catheter through the lumen of the body to the stenosis to be treated. At this location the balloon is inflated in order to expand the stent device to the required diameter in order to re-establish a sufficient blood passageway in the vessel. This expanded diameter will be somewhat larger than the natural diameter of the vessel in order to allow for natural tissue growth over the stent body without compromising the blood flow through the vessel. After expansion of the stent, the catheter is retracted leaving the implanted stent in position. Due to said tissue growth the stent will eventually lie substantially embedded in the vessel wall, while giving it continuous support.

Due to the helical pattern of the device, void of any discontinuities, and the great number of undulations within the structure, the overall device is extremely flexible both in the crimped as in the expanded state. Moreover the device of the invention is highly conformal to the natural course of the vessel to be treated and accordingly nicely fits to provide an evenly distributed support. The connection struts give the structure more coherence which results in an extremely well hoop strength, making the device especially suitable for the treatment of peripheral vessels which are relatively prone to sudden external loads.

Although the invention has been described hereinbefore with reference to merely a single embodiment it will be appreciated that the invention is by no means limited to this specific example. Within the scope of the invention many other embodiments and variations are on the contrary feasible for a skilled practitioner. As such he may use different amplitudes, pitches and patterns to tailor the device to a specific application. Also the number of helical windings of primary undulations may be larger than only a single one. Especially one or more parts of the device may comprise for instance two substantially continuous windings of mutually staggered primary undulations, advancing mutually substantial parallel along the longitudinal axis of said tubular member. A first one of those windings may then comprise said first and third undulation, while the other one comprises said second and fourth undulation. In case the connection elements are regularly distributed in between said windings and all mutually interconnected by connection struts the resulting, expanded structure will feature ring-like elements formed by the connection struts which are longitudinally interlinked by the helically advancing primary undulations. This structure is capable to a high conformability to the natural vessel course whilst having a large radial stiffness or hoop strength.

## Claims

1. An expandable intraluminal endoprosthesis comprising a tubular member having a first end and a second end and a wall surface (1) disposed between said first and second end, the wall having a first diameter in a first, unexpanded state which permits intraluminal delivery of the member into a lumen of a body passageway, particularly a blood vessel, and being capable of acquiring a second diameter in an expanded deformed state upon the exertion of a radially outwardly extending force to expand the lumen of the body passageway, at least part of said wall of said tubular member comprising at least one substantially continuous winding (10) of mutually staggered primary undulations (11-14) which advances substantially helically around a longitudinal axis of said tubular member, a first pair of primary undulations within immediately following subsequent turns of said winding being mutually connected by means of a first connection element (21), a second pair of primary undulations within said immediately following subsequent turns being mutually connected by means of a second connection element (22), ***characterized* in that** said first and second connection elements are mutually connected by means of a circumferentially orientated connection strut (30) lying interposed between said immediately following subsequent turns of said winding, at least in said first unexpanded state.

2. Endoprosthesis according to claim 1 **characterized in that** primary undulations (11-14) are mutually interconnected by means of a number of connections elements (21,22,23) which are regularly distributed over a helical turn of said at least one winding (10) and **in that** a connection strut (30) is present from each connection element (21,22,23) within said turn to a subsequent connection element (21,22,23) within said turn.

3. Endoprosthesis according to claim 2 **characterized in that** said connection strut (30) comprises a sub-structure of mutually staggered secondary undulations (31), at least in a crimped state of the device, lying in between subsequent turns of primary undulations (11-14).

4. Endoprosthesis according to claim 3 **characterized in that** said primary undulations (11-14) have a mutual pitch which is at least locally substantially an integer multiple of the mutual pitch of said secondary undulations.

5. Endoprosthesis according to claim 4 **characterized in that** the secondary undulations (31) have an amplitude substantial equal to half the longitudinal pitch of said turns.

6. Endoprosthesis according to claim 3, 4 or 5 **characterized in that** the connection elements comprise at least one tertiary undulation (20) which lies embedded in said substructure of mutually staggered secondary undulations (31) of said connection struts (30), at least in said first, unexpanded state of the device.

7. Endoprosthesis according to anyone of the preceding claims **characterized in that** at least a part of said wall of said tubular member comprises at least two substantially continuous windings of mutually staggered primary undulations (11-14), advancing mutually substantial parallel along the longitudinal axis of said tubular member, **in that** a first winding comprises a first and a third undulation (11,13), **in that** a second winding comprises a second and a fourth undulation (12,14), and **in that** the connection struts (30) are interposed in between both said windings.

8. Endoprosthesis according to anyone of the preceding claims **characterized in that** said structure comprises a substantial continuous filament which has been separated from a tube wall, **in that** said connection elements are an integral extension of the primary undulations which they interconnect and **in that** the connection strut is an integral extension of the connection elements thereby connected.

9. Endoprosthesis according to claim 8 **characterized in that** said connection strut exhibits a smaller filament width than the primary undulations.

## Patentansprüche

1. Aufweitbare intraluminale Endoprothese, umfassend ein röhrenförmiges Element mit einem ersten Ende und einem zweite Ende und einer Wandfläche (1), die zwischen dem ersten und zweiten Ende angeordnet ist, wobei die Wand einen ersten Durchmesser in einem ersten, nicht aufgeweiteten Zustand aufweist, der die intraluminale Zuführung des Elements in ein Lumen einer Körperpassage, insbesondere eines Blutgefäßes ermöglicht, und bei Anwendung einer radial nach außen gerichteten Kraft einen zweiten Durchmesser in einem aufgeweiteten, deformierten Zustand annehmen kann, so dass das Lumen der Körperpassage aufgeweitet wird, wobei wenigstens ein Teil der Wand des röhrenförmigen Elements wenigstens eine im wesentlichen ununterbrochene Windung (10) aus gegeneinander versetzten primären Wellenlinien (11-14) umfaßt, die im wesentlichen spiralförmig um die Längsachse des röhrenförmigen Elements verläuft, wobei ein erstes Paar primärer Wellenlinien innerhalb unmittelbar folgender, anschließender Drehungen der Windung mit Hilfe eines ersten Verbindungselements (21) miteinander verbunden ist, ein zweites Paar primärer Wellenlinien innerhalb der unmittelbar folgenden, anschließenden Drehungen mit Hilfe eines zweiten Verbindungselements (22) miteinander verbunden ist, **dadurch gekennzeichnet, dass** erstes und zweites Verbindungselement mit Hilfe einer bezüglich des Umfangs ausgerichteten Verbindungsstrebe (30) miteinander verbunden sind, die zumindest im ersten, nicht aufgeweiteten Zustand zwischen den unmittelbar folgenden, anschließenden Drehungen der Windung eingesetzt liegt.

2. Endoprothese nach Anspruch 1, **dadurch gekennzeichnet, dass** die primären Wellenlinien (11-14) mit Hilfe einer Reihe von Verbindungselementen (21, 22, 23) miteinander verbunden sind, die über eine spiralförmige Drehung der wenigstens einen Windung (10) regelmäßig verteilt sind, und dass eine Verbindungsstrebe (30) von jedem Verbindungselement (21, 22, 23) innerhalb der Drehung zu einem nachfolgenden Verbindungselement (21, 22, 23) innerhalb der Drehung vorhanden ist.

3. Endoprothese nach Anspruch 2, **dadurch gekennzeichnet, dass** die Verbindungsstrebe (30) eine Unterstruktur aus gegeneinander versetzten sekundären Wellenlinien (31) umfaßt, die zumindest im gefalteten Zustand der Vorrichtung zwischen aufeinanderfolgenden Drehungen der primären Wellenlinien (11-14) liegen.

4. Endoprothese nach Anspruch 3, **dadurch gekennzeichnet, dass** die primären Wellenlinien (11-14) eine gegenseitige Ganghöhe aufweisen, die zumindest lokal im wesentlichen ein ganzzahliges Vielfaches der gegenseitigen Ganghöhe der sekundären Wellenlinien ist.

5. Endoprothese nach Anspruch 4, **dadurch gekennzeichnet, dass** die sekundären Wellenlinien (31) eine Amplitude aufweisen, die im wesentlichen gleich der Hälfte der längsverlaufenden Ganghöhe der Drehungen ist.

6. Endoprothese nach Anspruch 3, 4 oder 5, **dadurch gekennzeichnet, dass** die Verbindungselemente wenigstens eine tertiäre Wellenlinie (20) umfassen, die zumindest im ersten, nicht aufgeweiteten Zustand der Vorrichtung eingebettet in die Unterstruktur der gegeneinander versetzten sekundären Wellenlinien (31) der Verbindungsstreben (30) liegt.

7. Endoprothese nach einem der vorstehenden Ansprüche, **dadurch gekennzeichnet, dass** wenigstens ein Teil der Wand des röhrenförmigen Elements wenigstens zwei im wesentlichen ununterbrochene Windungen aus gegeneinander versetzten primären Wellenlinien (11-14) umfaßt, die zueinander im wesentlichen parallel zur Längsachse des röhrenförmigen Elements verlaufen, dass eine erste Windung eine erste und eine dritte Wellenlinie (11, 13) umfaßt, dass eine zweite Windung eine zweite und eine vierte Wellenlinie (12, 14) umfaßt, und dass die Verbindungsstreben (30) zwischen diese beiden Windungen eingesetzt sind.

8. Endoprothese nach einem der vorstehenden Ansprüche, **dadurch gekennzeichnet, dass** die Struktur ein im wesentlichen ununterbrochenes Filament umfaßt, das von der Röhrenwand getrennt ist, dass die Verbindungselemente eine stoffschlüssige Verlängerung der primären Wellenlinien darstellen, die sie miteinander verbinden, und dass die Verbindungsstrebe eine stoffschlüssige Verlängerung der so verbundenen Verbindungselemente darstellt.

9. Endoprothese nach Anspruch 8, **dadurch gekennzeichnet, dass** die Verbindungsstrebe eine kleinere Filamentbreite als die primären Wellenlinien aufweist.

## Revendications

1. Une endoprothèse intraluminale extensible, comprenant un organe tubulaire, ayant une première extrémité et une deuxième extrémité, et une surface de paroi (1), disposée entre lesdites première et deuxième extrémités, la paroi ayant un premier diamètre en un premier état, non étendu, permettant une fourniture intraluminale de l'organe dans un lumen d'une voie de passage corporelle, en particulier un vaisseau sanguin, et étant susceptible d'acquérir un deuxième diamètre en un état déformé étendu, lors de l'exercice d'une force s'étendant radialement extérieurement, dans le but d'étendre le lumen de la voie de passage corporelle, au moins une partie de ladite paroi dudit organe tubulaire comprenant au moins un enroulement (10) sensiblement continu d'ondulations primaires (11 à 14) mutuellement en quinconce, progressant de façon sensiblement hélicoïdale autour d'un axe longitudinal dudit organe tubulaire, une première paire d'ondulations primaires, dans des spires subséquentes immédiatement suivantes dudit enroulement, étant mutuellement connectées au moyen d'un premier élément de connexion (21), une deuxième paire d'ondulations primaires, dans lesdites spires subséquentes immédiatement suivantes, étant mutuellement connectées au moyen d'un deuxième élément de connexion (22), **caractérisée en ce que** lesdits premier et deuxième éléments de connexion sont mutuellement connectés à l'aide d'un étai de connexion (30) orienté circonférentiellement, interposé entre lesdites spires subséquentes immédiatement suivantes dudit enroulement, au moins audit premier état non étendu.

2. Endoprothèse selon la revendication 1, **caractérisée en ce que** des ondulations primaires (11 et 14) sont mutuellement interconnectées à l'aide d'une pluralité d'éléments de connexion (21, 22, 23) qui sont répartis régulièrement sur une spire hélicoïdale dudit au moins un enroulement (10), et **en ce qu'**un étai de connexion (30) est présent depuis chaque élément de connexion (21, 22, 23) à l'intérieur de ladite spire, sur un élément de connexion (21, 22, 23) subséquent à l'intérieur de ladite spire.

3. Endoprothèse selon la revendication 2, **caractérisée en ce que** ledit étai de connexion (30) comprend une substructure, formée d'ondulations secondaires (31) mutuellement en quinconce, au moins en un état ondulé du dispositif, située entre des spires subséquentes des ondulations primaires (11 à 14).

4. Endoprothèse selon la revendication 3, **caractérisée en ce que** ladite pluralité d'ondulations (11 à 14) ont un pas mutuel qui est au moins localement sensiblement un multiple entier du pas mutuel desdites ondulations secondaires.

5. Endoprothèse selon la revendication 4, **caractérisée en ce que** les ondulations secondaires (31) ont une amplitude sensiblement égale à là moitié du pas longitudinal desdites spires.

6. Endoprothèse selon la revendication 3, 4 ou 5, **caractérisée en ce que** les éléments de connexion comprennent au moins une ondulation tertiaire (20) incorporée dans ladite substructure des ondulations secondaires (31) mutuellement en quinconce desdits étais de connexion (30), au moins audit premier état non étendu du dispositif.

7. Endoprothèse selon l'une quelconque des revendications précédentes, **caractérisée en ce qu'**au moins une partie de ladite paroi dudit organe tubulaire comprend au moins deux enroulements sensiblement continus d'ondulations primaires (11 à 14) mutuellement en quinconce ; progressant mutuellement sensiblement parallèlement selon l'axe longitudinal dudit organe tubulaire, **en ce qu'**un premier enroulement comprend une première et une troisième ondulations (11, 13), **en ce qu'**un deuxième enroulement comprend une deuxième et une quatrième ondulations (12, 14), et **en ce que** les étais de connexion (30) sont interposés entre lesdits enroulements.

8. Endoprothèse selon l'une quelconque des revendications précédentes, **caractérisée en ce que** ladite structure comprend un filament sensiblement continu, ayant été séparé d'une paroi de tube, **en ce que** lesdits éléments de connexion sont une extension monobloc des ondulations primaires qu'ils interconnectent, et **en ce que** l'étai de connexion est une extension monobloc des éléments de connexion ainsi connectés.

9. Endoprothèse selon la revendication 8, **caractérisée en ce que** ledit étai de connexion présente une largeur de filament inférieure auxdites premières ondulations.
